# EUROPÄISCHE PATENTSCHRIFT

(11) **EP 3 755 244 B1**
(45) Veröffentlichungstag und Bekanntmachung des Hinweises auf die Patenterteilung: **30.06.2021**
(21) Anmeldenummer: 20721528.6
(22) Anmeldetag: 23.04.2020
(51) Int. Cl.: A61B 17/15

(54) **AUSRICHTUNGSVORRICHTUNG MIT RÜCKMELDUNG**
ORIENTATION APPARATUS WITH FEEDBACK
DISPOSITIF D'ALIGNEMENT AVEC RETOUR D'INFORMATION

(30) Priorität: 16.05.2019 DE 102019112897
(43) Veröffentlichungstag der Anmeldung: 30.12.2020
(73) Patentinhaber: AESCULAP AG, 78532 Tuttlingen (DE)
(72) Erfinder: GASSNER, Stefan, 78194 Immendingen-Hattingen (DE); SCHÜLE, Jana, 88637 Kreenheinstetten (DE)
(74) Vertreter: Winter, Brandl - Partnerschaft mbB
(86) Internationale Anmeldenummer: PCT/EP2020/061292
(87) Internationale Veröffentlichungsnummer: WO 2020/229129

(56) Entgegenhaltungen:
- WO-A1-2010/029333
- US-A- 5 681 316
- US-A1- 2005 187 560
- US-A1- 2013 158 556

## Beschreibung

### Technisches Gebiet

Die vorliegende Erfindung betrifft eine Vorrichtung bzw. eine Ausrichthilfe zum Ausrichten einer Sägeschablone bzw. Sägeblock zur Entfernung eines Bereichs des Schienbeinkopfes bei der Implantation einer Kniegelenkprothese (nachfolgend auch Tibia-Ausrichthilfe genannt).

### Hintergrund der Erfindung

Bei der Implantation beispielsweise eines künstlichen Kniegelenkes muss ein Chirurg unter anderem den defekten Kopfteil des tibialen Kniegelenks (Schienbeinkopf, Tibiakopf) mit einer oszillierenden Knochensäge entfernen. Dazu verwendet der Chirurg eine Ausrichtvorrichtung oder Ausrichthilfe, welche ihm ermöglicht, eine Sägeschablone (auch als Sägeblock bezeichnet) bezüglich der Tibia in der richtigen Höhe und in der korrekten Winkelstellung am Tibiakopf zu befestigen.

Am Beginn einer Implantation wird die Schablone durch eine extramedulläre oder intramedulläre Haltevorrichtung (diese hat oder ist die Ausrichthilfe) an dem Tibiakopf gehalten. In anderen Worten kann die Halterung der Schablone extramedullär, also durch eine Halterung die extern an dem Bein bzw. an dem Schienbein angebracht ist, oder intramedullär, also durch einen Knochen- bzw. Marknagel oder dergleichen, der in den Schienbeinknochen eingebracht wird, an dem Tibiakopf gehalten werden. Sobald die Schablone mittels der extramedulläre oder der intramedulläre Haltevorrichtungen an der Tibia gehalten ist, erfolgt die Feineinstellung der Relativlage und Ausrichtung der Schablone mittels eines Stylus (ist die Ausrichthilfe und ggf. Bestandteil der Haltevorrichtung). Der Stylus ermittelt die richtige Höhe der Schablone relativ zum Tibiakopf an dem Knochen selbst. Sobald die Höhe gefunden ist, wird die Schablone mittels Schrauben an den Tibiakopf festgeschraubt und die Haltevorrichtung einschließlich Stylus wird wieder entfernt.

Um die künstliche Tibiakomponente des künstlichen Gelenks auf der ursprünglichen Gelenklinie zu positionieren, muss der Arzt mit Hilfe des Stylus die tiefste Defektstelle im Knochen antasten und die Dicke des zu entfernenden Knochenstückes mittels eines Drehrads und einer Skala einstellen. Der einzustellende Wert entspricht in der Regel der herstellerspezifischen Implantatdicke, wodurch die ursprüngliche Gelenklinie wieder hergestellt wird. Aus medizinischer Sicht kann es jedoch notwendig sein, von der ursprünglichen Gelenklinie abzuweichen und diese gezielt nach zum Körper hin/ in Richtung distal oder vom Körper weg/ in Richtung proximal zu verschieben.

Eine Knieprothese, Knieendoprothese, künstliches Kniegelenke oder Kniegelenksprothese ist eine implantierte Prothese (Endoprothese), die das Kniegelenk ganz oder teilweise ersetzt. Die Knieprothese wird hauptsächlich bei schwerem Verschleiß des Knies (Kniegelenksarthrose) und nach Verletzungen des Knies als operative Therapie eingesetzt, um die schmerzfreie Bewegungsfähigkeit und ggf. bei Bandinstabilitäten zusätzlich auch die Kniegelenksstabilität wiederherzustellen. Es können Teil- oder Totalendoprothesen verwendet werden.

Das Schienbein (Tibia) ist neben dem Wadenbein (Fibula) einer der beiden Knochen des Unterschenkels. Das Schienbein ist der kräftigere der beiden Knochen und ein typischer Röhrenknochen. Am kräftigsten ausgebildet ist das obere Ende, der Kopf (Caput tibiae), welcher zwei Gelenkknorren trägt (Condylus medialis und Condylus lateralis). Als Knorren oder Kondylus oder Knöchel bezeichnet man in der Anatomie den knöchernen Teil eines Gelenks, der auch als Gelenkfortsatz bezeichnet wird. Im Kniegelenk tragen das obere Ende des Schienbeins (Tibia) und das untere Ende des Oberschenkelknochens (Femur) jeweils zwei Gelenkknorren. Diese besitzen auf ihrer oberen Fläche eine überknorpelte Gelenkfläche (Facies articularis superior), die durch eine Erhöhung (Eminentia intercondylaris) in zwei Anteile getrennt wird. Die Erhöhung läuft in zwei getrennte Höckerchen (Tuberculum intercondylare mediale und Tuberculum intercondylare laterale) aus. Begrenzt wird sie vorne (ventral) und hinten (dorsal) durch zwei flache Gruben (Area intercondylaris anterior und Area intercondylaris posterior). Dort setzen die Kreuzbänder und die Haltebänder der Menisken an. Die gesamte obere Fläche des Schienbeines wird als Schienbeinplateau bezeichnet und bildet mit den Knorren des Oberschenkelknochens (Femur) das Kniegelenk. Am seitlichen Umfang des nahezu senkrecht stehenden Knochenrandes findet sich die Gelenkfläche (Facies articularis fibularis) für den Wadenbeinkopf (Caput fibulae).

Die Tibiaresektion kann wahlweise mit der extramedullären oder der intramedullären Tibiaresektionslehre erfolgen. Jede der beiden Techniken bietet verschiedene Einstellmöglichkeiten für unterschiedliche anatomische Verhältnisse und Arbeitsweisen. Extramedullär bedeutet "außerhalb des Markes" gelegen und bezieht sich auf das Knochenmark (Medulla ossium) oder das Rückenmark (Medulla spinalis). Intramedullär bedeutet "innerhalb des Markes" gelegen und bezieht sich auf das Knochenmark (Medulla ossium) oder das Rückenmark (Medulla spinalis).

### Stand der Technik

Viele Ärzte präparieren die Tibia in zwei Schritten, um dazwischen eine Kontrolle des präparierten Knochens durchzuführen. In einem ersten Schritt werden nur 2 mm entfernt und beispielsweise in einem zweiten Schritt weitere 6 mm, so dass insgesamt 8mm entfernt werden. Einige Chirurgen vertreten die Ansicht, immer möglichst knochensparend zu operieren und entfernen daher so wenig Knochen wie möglich. In diesem Fall wird eine Verschiebung der Gelenklinie bewusst akzeptiert, oder aber durch die Auswahl von entsprechenden Implantatdicken kompensiert.

Die Kenntnis der exakten Dicke des entfernten Knochenteils ist in jedem Fall für den weiteren Operationsverlauf relevant. Zu diesem Zweck hat oder besteht eine aus dem Stand der Technik bekannte Ausrichtvorrichtung oder Ausrichthilfe für eine tibiale Resektionsführung aus einer Antastvorrichtung, die ein in der Regel wendeiförmiges Verstellelement aufweist, an dem ein, eine proximale Antastspitze aufweisender Tastbalken gehalten ist. Ferner ist ein in der Regel wendelradförmiges Einstellelement vorgesehen, welches mit dem Verstellelement, d.h. der Wendel, in Wirkeingriff ist, sodass durch manuelle Betätigung des Wendelrads der Tastbalken längs der Wendel bewegt werden kann. Schließlich ist ein am Tastbalken oder am Wendelrad fixiertes/gehaltenes sowie längs der Wendel sich erstreckender Skalenträger mit einer darauf angebrachten Skala vorgesehen, der/die mit Referenz auf eine Markierung am Wendelrad oder an der Wendel eine aktuelle Position der Antastspitze längs der Wendel angibt. Ist somit die derart aufgebaute Ausrichtvorrichtung mit der Sägeschablone gekoppelt, kann der Relativabstand zwischen der Sägeschablone und der Antastspitze längs der Wendel verstellt/feinjustiert werden.

WO 2010/029333 A1 offenbart eine chirurgische Resektionsführung für eine Tibia mit einem Gehäuse, einem sich durch das Gehäuse erstreckenden Tastarm, der so angeordnet ist, dass wenn das Gehäuse an die Seite eines Knochens gekoppelt ist, eine Spitze des Tastarms angeordnet ist, um ein Ende eines Knochens zu kontaktieren, und einem linearen Einstellmechanismus.

Ferner betrifft die US 2005/0187560 A1 eine Vorrichtung und ein zugehöriges Verfahren zum Ausmessen eines distalen Femurs mit einer Basis, die so eingerichtet ist, dass sie gleichzeitig an einer distalen Oberfläche und einer hinteren Oberfläche des distalen Femurs anliegt, und ferner mit einer axialen Abstandsskala.

Ferner offenbart die US 2013/0158556 A1 ein chirurgisches Schneidwerkzeug mit einem Resektionsturm und einer mit diesem koppelbaren Valgusführung mit einem drehbaren Element. Das drehbare Element kann eine eckige Oberfläche mit einem oder mehreren Keilen mit variabler Tiefe aufweisen, die eine Varus- / Valgus-Winkeleinstellung des Schneidblocks ermöglichen.

Bei der Ermittlung der Dicke des entfernten bzw. zu entfernenden Knochenteils ist die Einstell- und Ablesegenauigkeit der Skala entscheidend. Im Stand der Technik sind diese beiden Faktoren jedoch begrenzt. Die Skala bietet im Stand der Technik zwar eine stufenlose Einteilung in Millimeter-Schritten über ein Gewinde (Wirkeingriff zwischen Wendel und Wendelrad). Während der Anwendung ist jedoch die Blickrichtung des Chirurgen nicht parallel zu der Skala, sondern in einem Winkel von ca. 45° von oben auf eine Mittelachse des Stylus/Ausrichthilfe. Die Mittelachse ist die Achse, um die das Wendelrad (Einstellrad/ Drehrad) des Stylus rotiert. Dadurch können im Stand der Technik Parallaxenfehler (Beobachtungsfehler) entstehen. Zudem bietet die stufenlose Einstellbarkeit Interpretationsspielraum, sobald ein Wert zwischen zwei Markierungen eingestellt wird. Außerdem kann die Höhe durch unbeabsichtigte Berührung des Wendel-/Drehrades verstellt werden, da keine Feststellung gegeben ist.

### Kurzbeschreibung der Erfindung

Aus diesem Grund besteht die Notwendigkeit und damit die Aufgabe der vorliegenden Erfindung eine genaue Einstellbarkeit der Ausrichtungsvorrichtung und eine gute Ablesbarkeit bzw. Erfassbarkeit der Skala insbesondere der aktuellen Position der Abtastspitze an dem Verstellelement bereitzustellen.

Die Aufgabe wird durch eine Ausrichthilfe oder Ausrichtungs-/Antastvorrichtung gemäß den Merkmalen des Patentanspruches 1 gelöst. Vorteilhafte Ausgestaltungen und/oder Weiterbildungen der vorliegenden Erfindung sind Gegenstand der Unteransprüche.

Der Kerngedanke der vorliegenden Erfindung besteht im Wesentlichen darin, dem Chirurgen bei der Verwendung, d.h. bei der Einstellung/Justierung der erfindungsgemäßen Ausrichthilfe/Antastvorrichtung ein taktiles und/oder akustisches Signal zu geben/vermitteln, aus welchem der Chirurg Rückschlüsse über die Position des Tasters/der Tastspitze längs des Verstellelements (z.B. Wendel) bzw. die Distanz zwischen Taster und Sägeschablone ziehen kann, ohne dass ein visuelles Abgreifen des aktuellen Skalenwerts (per Auge) zusätzlich erforderlich ist. Konstruktiv kann dies dadurch erreicht werden, indem das Einstellelement (z.B. Drehrad/Wendelrad) mit zumindest einem Vor- und/oder Rücksprung (erstes Anschlagteil) ausgebildet ist, welches in Abhängigkeit der (immer wieder sich wiederholenden/durchwandernden) Einstellposition im Verlauf der Einstellbewegung des Einstellelements (z.B. Drehung des Wendelrads) mit einem (bzgl. der Einstellbewegung des Einstellelements ortsfesten) Zeiger/Schapper (zweites Anschlagteil) in (Rast-/Anschlags-)Anlage bringbar ist, wodurch der vom Einstellelement erzeugte Widerstand (Widerstandskraft) gegen die manuell aufgebrachte Einstellbetätigungskraft verändert (erhöht) wird. Auf diese Weise kann ein Chirurg die aktuelle Relativlage (z.B. Drehwinkel) zwischen Einstellelement (z.B. Drehrad) und dem Zeiger/Schnapper an zumindest einer Stelle im Verlauf einer Einstellbewegung des Einstellelements erfühlen /ertasten und damit auf die Position/den Verstellweg des Tasters/der Tastspitze am Verstellelement rückschließen.

Handelt es sich bei dem ersten Anschlagteil um einen nockenförmigen Vorsprung, wird der Zeiger/Schnapper bei einer Weiterbetätigung des Einstellelements elastisch vom nockenförmigen Vorsprung weggedrückt (verdrängt). Sobald der Zeiger/Schnapper bei entsprechender Weiterbetätigung des Einstellelements das Ende des nockenförmigen Vorsprungs erreicht hat, schnappt dieser (bei entsprechender Formgebung des Vorsprungs) schlagartig zurück, was durch ein ggf. lautes Klacken hörbar ist. Auf diese Weise lassen sich wenigstens eine, vorzugsweise zwei (immer wieder sich wiederholende/durchwandernde) Einstellpositionen des Einstellelements ertastet und/oder erhört werden. Dabei sei darauf hingewiesen, dass anstelle oder zusätzlich zu dem genannten Zeiger/Schnapper auch der nockenförmige Vorsprung elastisch nachgeben kann.

Gemäß einem ersten Aspekt der Erfindung hat oder ist die Ausrichtungsvorrichtung/Ausrichthilfe zum Ausrichten einer Sägeschablone eine Antastvorrichtung mit einem Taster, dessen Abstand zur Sägeschablone einstellbar ist, wofür folgendes vorgesehen ist:
ein bewegbar, vorzugsweise drehbar gestaltetes Einstellmittel/Einstellelement (z.B. Wendelrad oder Zahnrad) ,
ein Verstellmittel/Verstellelement mit einer profilierten Einkerbung (z.B. Wendel oder Zahnstange), das mit dem Einstellmittel in (Wirk-)Eingriff ist und durch Bewegung/Betätigung des Einstellmittels ebenfalls bewegbar ist,
ein, eine Skala aufweisender Skalenträger zum Messen eines Verstellwegs des Tasters vorzugsweise relativ zur Sägeschablone in Verstellmittel-Längsrichtung,
wenigstens ein erstes Anschlagmittel/Anschlagteil, das an dem Einstellmittel ausgeformt ist,
wenigstens ein zweites Anschlagmittel/Anschlagteil, das an dem Skalenträger ausgeformt ist oder als Skalenträger dient und das vorgesehen und angepasst ist, als Widerstand für das erste Anschlagmittel bei einer Bewegung des Einstellmittels um einen bestimmtem Bewegungsbetrag oder Bewegungsweg zu fungieren, wobei
das zweite Anschlagmittel oder das erste Anschlagmittel das jeweils andere Anschlagmittel bei einer Weiterbewegung des Einstellmittels über den bestimmten Bewegungsbetrag oder Bewegungsweg hinaus federnd auslenkt.

In bevorzugter Weise ist die erfindungsgemäße Ausrichtungsvorrichtung ein Stylus/Antastvorrichtung selbst, die dafür vorgesehen und angepasst ist, mit einer Sägeschablone gekoppelt zu werden oder mit dieser gekoppelt ist, um die Sägeschablone an einem Knochen, vorzugsweise einem Tibiakopf, zu positionieren. Die Antastvorrichtung gemäß der Erfindung kann mittels einer extramedullären oder einer intramedullären Haltevorrichtung positioniert werden oder sie ist Teil der Haltevorrichtung.

In einer bevorzugten Ausführungsform bewegt sich das Verstellmittel relativ zu dem Einstellmittel. Das Verstellmittel ist vorzugsweise ein Zylinder/Stange mit Gewinde insbesondere ein Außengewinde bzw. Wendel oder eine Oberfläche mit Zähnen oder ein Zylinder mit Zähnen - also eine Zahnstange - oder ein Zylinder mit profilierten Einkerbungen oder dergleichen.

Das Einstellmittel ist vorzugsweise ein Rad insbesondere ein mit dem Verstellmittel in (Wirk-)Eingriff stehendes Drehrad oder Wendelrad oder ein federvorgespannter Verstellkopf oder dergleichen ggf. mit einem Gewinde vorzugsweise einem Innengewinde oder ein Zahnrad bzw. ein Rad mit über den Umfang gleichmäßig verteilten Zähnen oder profilierten Einkerbungen oder dergleichen. Das Einstellmittel verstellt bei dessen manueller Betätigung den Taster vorzugsweise entlang einer Längsachse des Verstellmittels (Wendel/Drehrad-Kombination) oder senkrecht zu einer Drehachse des Einstellmittels (Zahnradstange/Zahnrad-Kombination).

Mit dem Verstellmittel ist nach einer bevorzugten Ausgestaltung der Taster vorzugsweise bestehend aus einem Tastbalken mit einer proximal daran angeordneten Tastspitze und vorzugsweisedem Skalenträger mit daran befestigter oder aufgedruckter Skala fest verbunden oder einteilig ausgebildet oder angeordnet, um so zusammen mit dem Verstellmittel bewegt werden zu können. Nach einer alternativen Ausgestaltung ist es auch möglich, den Taster beweglich am Verstellmittel zu halten und diesen über das Einstellmittel für eine Bewegung des Verstellmittels in Längsrichtung des Verstellmittels zu bewegen. Das Verstellmittel bewegt sich somit gemäß einer ersten Alternative relativ zu dem Einstellmittel oder gemäß einer zweiten Alternative zusammen mit dem Einstellmittel, wobei die Skala bzw. der Skalenträger (der vorzugsweise mit dem Tastbalken fest verbunden ist) sich grundsätzlich relativ zu dem Einstellmittel bewegt. Die Skala kann somit einen relativen Wert oder Abstand oder Distanz anzeigen, an dem sich der Taster relativ zu dem Einstellmittel befindet.

Im Konkreten kann das Verstellmittel ein Hohlzylinder mit wendelartigem Außengewinde sein, der auf eine mit der Sägeschablone verbundene/verbindbare innere Achse drehfest aber axialverschiebbar aufgesteckt ist und das Einstellmittel ein am freien Ende der inneren Achse drehgelagertes (axialfestes) Wendelrad mit Innengewinde sein, die mit dem Außengewinde des Hohlzylinders in Wirk-/Schraubeingriff steht und bei einer (manuellen) Drehbewegung den Hohlzylinder längs der (inneren) Achse verschiebt. In diesem Fall zeigt die Skala/der Skalenträger, der am mit dem Hohlzylinder fest verbundenen Taster oder am Hohlzylinder selbstbefestigt ist den Schiebeweg/Distanz des Hohlzylinders und damit des Tasters zum Wendelrad an und somit indirekt die Distanz zwischen Schablone und Taster.

An dem Einstellmittel ist vorzugsweise das erste Anschlagsmittel ausgeformt. Das erste Anschlagsmittel hat vorzugsweise die Form einer Nut, einer Auskerbung oder einer Aussparung oder eines Vorsprungs, eines Zahnes, eines Federdrahtes, eines Federplättchens oder einer Nase oder dergleichen. An dem Einstellmittel können eines oder mehrere dieser ersten Anschlagsmittel ausgeformt sein, vorzugsweise in einem bestimmten Winkelabstand zum Beispiel von 180° zueinander. Bei einem Rad/Drehknopf als Einstellmittel dreht sich bei jeder Drehung des Rades das wenigstens eine Anschlagmittel mit. Bei einem federvorgespannten Verstellkopf als Einstellmittel drückt eine federvorgespannte Kugel oder dergleichen in dem Kopf gegen das Verstellmittel.

An der Skala bzw. am Skalenträger ist das zweite Anschlagmittel ausgeformt oder der Skalenträger bildet das zweite Anschlagmittel. Das zweite Anschlagsmittel hat vorzugsweise die Form einer Nut, einer Auskerbung oder einer Aussparung oder eines Vorsprungs, eines Zahnes, eines Federdrahtes, eines Federplättchens oder einer Nase oder dergleichen. Das zweite Anschlagmittel ist vorgesehen und angepasst als, vorzugsweise rastender Widerstand für das erste Anschlagmittel zu fungieren. In anderen Worten ausgedrückt, ist das zweite Anschlagmittel so ausgeformt, dass es in Kontakt mit dem ersten Anschlagmittel kommt und dabei in einen rastenden rastbaren Eingriff mit diesem kommt oder gelangt.

Das erste und/oder das zweite Anschlagmittel sind federnd gelagert oder auf einer federnden Lagerung ausgeformt oder auf einem federelastischen Bauteil ausgeformt oder auf einer/einem federelastischen Skala/Skalenträger ausgeformt oder bildet ein federelastisches Bauteil oder bildet eine federelastische Skala. In anderen Worten ausgedrückt kommt beim Bewegen des Einstellelements ein (erstes) Anschlagsmittel in Kontakt oder Eingriff mit dem anderen (zweiten) Anschlagmittel. Dabei kann das erste und/oder das zweite Anschlagmittel federnd gelagert sein. Das bedeutet, dass ein Anschlagmittel gegen die federnde Lagerung oder Skala des anderen Anschlagsmittels auf dem dieses ausgebildet ist oder das andere Anschlagmittel selbst drückt. Das eine Anschlagmittel drückt diese federnde Lagerung oder Skala oder das federnde Anschlagmittel (radial) weg von dem Einstellmittel und die Anschlagmittel rasten ineinander ein oder stoßen gegeneinander. Bei einem weiteren Bewegen des Einstellmittels löst sich das eine Anschlagmittel aus dem Rasteingriff des anderen Anschlagmittels, indem die federnde Lagerung oder Skala oder das andere Anschlagmittel selbst durch das eine Anschlagmittel von dem Einstellmittel weg gedrückt wird. Dabei kann die Lagerung oder Skala oder ein Anschlagmittel selbst wenigstens ein Federelement oder federelastisch sein oder mehrere Federelemente in Reihe oder parallel aufweisen, so dass bei einer Bewegung des Einstellmittels ein Anschlagmittel bei Zurücklegen einer vorbestimmten Distanz immer wieder in Kontakt mit dem anderen Anschlagmittel kommt. Das erste und das zweite Anschlagmittel können aus allen federnden Materialien hergestellt werden (z.B. Federstahl, Kunststoffen oder Verbundmaterialien). Die Auslenkung der Anschlagmittel kann nicht nur radial zu einer Drehachse des Einstellmittels erfolgen, sondern auch in Rotationsrichtung um die Drehachse.

Wenn ein Anschlagmittel in das andere Anschlagmittel einrastet, gibt die Ausrichtungsvorrichtung beim Einstellen ein haptisches, taktiles, akustisches (ein deutlich hörbares Klicken) und/oder sichtbares Feedback oder Signal an den Benutzer. Die Erfindung kann somit bei einer Drehung des Einstellmittels das Verstellmittel eine vorbestimmte Distanz bewegen lassen und nach dieser Distanz ein Signal verursachen. Die Rastfunktion stellt gleichzeitig die Sicherung gegen versehentliches Verstellen dar. Somit wird eine akustische und/oder taktile Rückmeldung zur einfachen und schnellen Kontrolle des eingestellten Wertes bereitgestellt und eine Sicherung des eingestellten Wertes bereitgestellt, ohne die Baugröße des Stylus (Antasthilfe) dadurch maßgeblich zu verändern. Der Benutzer (z.B. Arzt oder Chirurg) kann ausgehend von einem oberen (0mm) zu einem unteren (12mm) Endanschlag den einzustellenden Wert schnell und zuverlässig anhand der Anzahl der "Klicks" (Einrastungen) ermitteln, wobei das obere Ende in Richtung hin zum Einstellmittel liegt und das untere Ende in Richtung hin zur Sägeschablone oder zum Tasterhalter. Ablesefehler können somit vermieden, eine Erhöhung der Sicherheit in der Anwendung gewährleistet und ein Zeitgewinn bei optimaler Flexibilität erreicht werden. Das Sichtfeld auf den Knochen wird bei dieser Lösung nicht zusätzlich eingeschränkt.

Vorzugsweise ist die Ausrichtungsvorrichtung so ausgestaltet, dass der Kraftaufwand über den gesamten Einstellbereich der Skala, also über den ganzen Bereich, in dem ein Anschlagmittel in das anderen Anschlagmittel einrastet, zwar annähernd konstant ist aber ein Anschlagswiderstand bewirkt wird, der vom Arzt/Chirurgen erfühlbar ist. Ebenso wird eine schlanke Bauform der Skala dadurch erhalten.

Alle Ausführungsformen der Erfindung haben den gleichen Kerngedanken und können sinngemäß miteinander kombiniert werden. So kann ein erstes Anschlagmittel einer Ausführungsform mit einem Anschlagmittel einer anderen Ausführungsform kombiniert werden, um ein akustisches, taktiles und/oder optisches Feedback zu erhalten.

### Figurenbeschreibung

Die Erfindung wird nachstehend anhand eines bevorzugten Ausführungsbeispiels unter Bezugnahme auf die begleitenden Zeichnungen näher erläutert. Es zeigen
- Fig. 1: eine Ausrichtungsvorrichtung/Antastvorrichtung auf einem extramedullären Gestell/Haltevorrichtung ,
- Fig. 2: eine ausgerichtete Sägeschablone an einem Tibiakopf,
- Fig. 3: eine Ausrichtungsvorrichtung/Antastvorrichtung,
- Fig. 4: eine erste Ausführungsform einer Ausrichtungsvorrichtung/Antastvorrichtung gemäß der Erfindung in einer Rückansicht,
- Fig. 5: die erste Ausführungsform der Ausrichtungsvorrichtung gemäß der Erfindung in einer Frontansicht,
- Fig. 6: die erste Ausführungsform der Ausrichtungsvorrichtung gemäß der Erfindung in einer Detailansicht,
- Fig. 7: die erste Ausführungsform der Ausrichtungsvorrichtung gemäß der Erfindung in einer Schnittansicht in einer ersten Position,
- Fig. 8: die erste Ausführungsform der Ausrichtungsvorrichtung gemäß der Erfindung in einer Schnittansicht in einer zweiten Position,
- Fig. 9: eine zweite Ausführungsform einer Ausrichtungsvorrichtung/Antastvorrichtung gemäß der Erfindung,
- Fig. 10: eine dritte Ausführungsform einer Ausrichtungsvorrichtung/Antastvorrichtung gemäß der Erfindung,
- Fig. 11: eine vierte Ausführungsform einer Ausrichtungsvorrichtung/Antastvorrichtung gemäß der Erfindung,
- Fig. 12: eine fünfte Ausführungsform einer Ausrichtungsvorrichtung/Antastvorrichtung gemäß der Erfindung,
- Fig. 13: eine sechste Ausführungsform einer Ausrichtungsvorrichtung/Antastvorrichtung gemäß der Erfindung,
- Fig. 14: eine siebte Ausführungsform einer Ausrichtungsvorrichtung/Antastvorrichtung gemäß der Erfindung,
- Fig. 15: eine achte Ausführungsform einer Ausrichtungsvorrichtung/Antastvorrichtung gemäß der Erfindung,
- Fig. 16: eine neunte Ausführungsform einer Ausrichtungsvorrichtung/Antastvorrichtung gemäß der Erfindung,
- Fig. 17: eine zehnte Ausführungsform einer Ausrichtungsvorrichtung/Antastvorrichtung gemäß der Erfindung,
- Fig. 18: eine elfte Ausführungsform einer Ausrichtungsvorrichtung/Antastvorrichtung gemäß der Erfindung mit seitlicher Belastung,
- Fig. 19: die elfte Ausführungsform einer Ausrichtungsvorrichtung gemäß der Erfindung mit frontaler Belastung,
- Fig. 20: eine zwölfte Ausführungsform einer Ausrichtungsvorrichtung/Antastvorrichtung gemäß der Erfindung mit frontaler Belastung,
- Fig. 21: eine dreizehnte Ausführungsform einer Ausrichtungsvorrichtung/Antastvorrichtung gemäß der Erfindung in Fronansicht,
- Fig. 22: die dreizehnte Ausführungsform einer Ausrichtungsvorrichtung/Antastvorrichtung gemäß der Erfindung in Rückansicht,
- Fig. 23: eine vierzehnte Ausführungsform einer Ausrichtungsvorrichtung gemäß der Erfindung,
- Fig. 24: eine fünfzehnte Ausführungsform einer Ausrichtungsvorrichtung/Antastvorrichtung gemäß der Erfindung,
- Fig. 25: eine sechzehnte Ausführungsform einer Ausrichtungsvorrichtung/Antastvorrichtung gemäß der Erfindung,
- Fig. 26: eine siebzehnte Ausführungsform einer Ausrichtungsvorrichtung/Antastvorrichtung gemäß der Erfindung.

### Beschreibung der Ausführungsbeispiele

### Nachstehend werden Ausführungsbeispiele der vorliegenden Offenbarung auf der Basis der zugehörigen Figuren beschrieben.

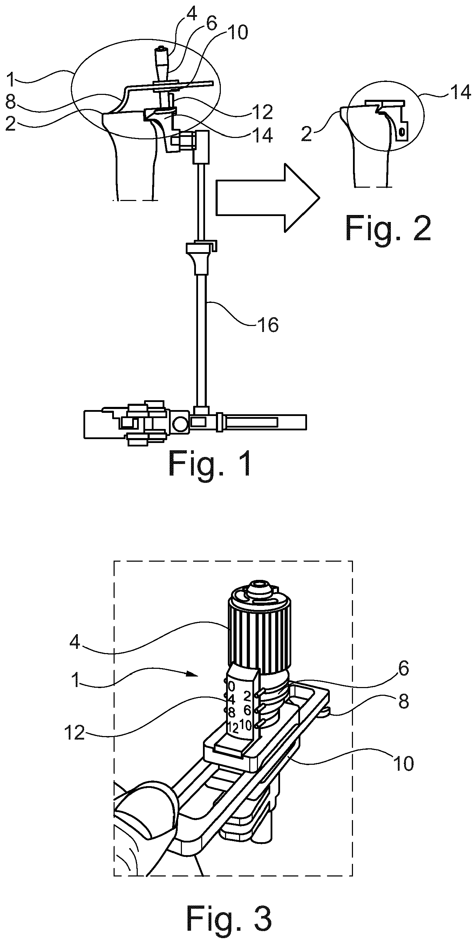

Fig. 1 zeigt prinzipiell ein mögliches Beispiel für eine extramedulläre Ausrichtungsvorrichtung/Haltevorrichtung für eine tibiale Resektionsführung, wie sie auch beim vorliegenden Erfindungsgegenstand vorgesehen sein kann. Diese besteht/hat beispielhaft (nicht darauf beschränkend) eine Teleskopstange 16, eine Fußfessel, die am distalen Ende der Telekopstange 16 befestigt ist, eine Sägeschablone/Sägeblock 14, die am proximalen Ende der Teleskopstange 16 fest (unlösbar) befestigt ist und eine proximale Ausricht-/Antastvorrichtung (Stylus) 1, die bevorzugt an/auf der Sägeschablone 14 montiert ist.

Die Ausricht-/Antastvorrichtung 1 wird an einem Tibiakopf verwendet. Sie hat in der Regel einen Taster 8, ein Verstellmittel 6 zu Verstellung einer Distanz zwischen Taster 8 und Sägeschablone 14 sowie ein Einstellmittel 4 zur Betätigung des Verstellmittels 6.

Das Einstellmittel 4, beispielsweise in Form eines Drehrades, verstellt/betätigt durch dessen manuelle Rotation das Verstellmittel 6, beispielsweise in Form eines Zylinders mit Außengewinde, d.h. eine Wendel. Der Taster 8 hat bevorzugt eine proximale Tastspitze, die über einen Tastbalken iin/an einem Tasterhalter 10 eingebracht/gelagert ist und über diesen fest oder (alternativ) beweglich, d.h. über das Außengewinde mit dem Verstellmittel 6 verbunden ist. Dies bedeutet in beiden Fällen, dass wenn das Einstellmittel 4 betätigt wird, bewegt sich der Tastenhalter 10 und damit der Taster 8 entsprechend dem Betätigungsbetrag sowie der Betätigungsrichtung in Richtung hin zum oder weg vom Einstellmittel 4. Zudem ist der Taster 8 verschiebbar in dem Tastenhalter 10 gelagert. Eine Skala bzw. ein Skalenträger 12 zeigt die Distanz oder den Abstand oder einen relativen Wert des Tasters 8 relativ zu dem Einstellmittel 4 an, um eine Sägeschablone 14 richtig an dem Tibiakopf 2 zu positionieren. In anderen Worten ausgedrückt, ist der Abstand des Einstellmittels 4 zur Sägeschablone 14 in montiertem Zustand bekannt und fix. Somit kann über die zwischen dem Einstellmittel 4 und dem Taster 8 sich befindliche Skala 12 der aktuelle Abstand des Tasters 8 zur Sägeschablone 14 angezeigt werden.

Fig. 2 zeigt die ausgerichtete Sägeschablone 14 an einem Tibiakopf 2, wobei in diesem Fall die Ausricht-/Antastvorrichtung noch nicht an der Sägeschablone montiert ist

Fig. 3 zeigt die Ausrichtungs-/Antastvorrichtung 1 ohne Sägeschablone nochmals in einer anderen Ansicht. Das drehbar sowie axialfest an einem inneren Haltebolzen/Achse gelagerte Drehrad 4 bewegt das Verstellmittel 6, d.h. den auf den inneren Haltebolzen axial verschiebbar aufgesteckten (Hohl-)Zylinder mit dem Außengewinde (Wendel) 6 an dem der Taster 8 mittels eines Tasterhalters 10 gehalten/fixiert ist in Axialrichtung, wodurch auch der Tasterhalter 10 in Axialrichtung längs des inneren Haltebolzens bewegt wird. Die Skala/Skalenträger 12 ist mit Tasterhalter oder dem Hohlzylinder 6 fest verbunden. Das Drehrad 4 zieht oder drückt den Hohlzylinder (mit Außengewinde) 6 somit zu dem Drehrad 4 nach oben (proximal) hin oder schiebt es von dem Drehrad 4 nach unten (distal) weg. Um das Drehrad 4 besser greifen zu können, hat dieses einen Axialabschnitt mit einer Außenrändelung/Riffelung oder Außengripfläche, wie dies insbesondere in Fig. 3 dargestellt ist.

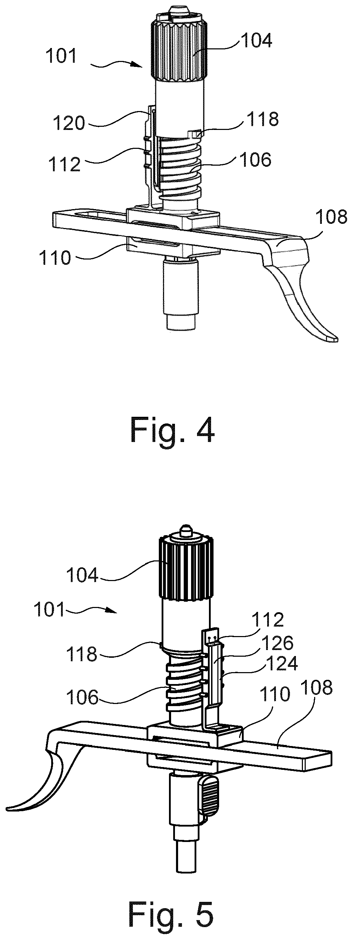

Fig. 4 zeigt nun im Konkreten eine erste Ausführungsform einer Ausrichtungsvorrichtung 101 gemäß der Erfindung in einer Rückansicht, welche auf der zuvor prinzipiell beschriebenen Ausrichtungsvorrichtung 1 aufbaut. Die Ausrichtungsvorrichtung 101 weist das Einstellmittel 104 in Form des um den inneren (drehfesten) Haltebolzen axialfest sowie drehbar gelagerten Drehrades auf, das durch Rotation an den Haltebolzen das Verstellmittel 106 in Form des (Hohl-)Zylinders mit Außengewinde (Wendel) längs des Haltebolzens höhenverstellt. Der Taster 108 ist dabei über den Tasterhalter 110 fest mit dem Verstellmittel 106 gekoppelt/verbunden. Die Skala bzw. der Skalenträger 112 ist vorliegend am Tasterhalter 110 befestigt, ragt in Richtung hin zu dem Einstellmittel 104 und zeigt somit die Höhe/Distanz des Tasters 108 relativ zu dem Einstellmittel 104 an, um die Sägeschablone (in Fig. 4 nicht dargestellt) richtig an einem Tibiakopf zu positionieren. Der Taster 108 in dem Tasterhalter 110 an dem Verstellmittel 106 und die Skala 112 auf/an dem Tasterhalter 110 bewegen sich somit bei einer Drehung/Betätigung des Einstellmittels (Drehrad) 104 relativ zu dem Einstellmittel 104. Soweit ist der Aufbau der Erfindung analog zu der Ausrichtungsvorrichtung aus den vorstehenden Figuren.

Das Einstellmittel 104 der Erfindung hat ebenfalls den Axialabschnitt mit einer aufgerauten/geriffelten Außenfläche, an die sich distal (in Richtung hin zum Tasterhalter) erfindungsgemäß ein Axialabschnitt mit vergleichsweise glatter Außenfläche anschließt, die dafür vorgesehen und ausgebildet ist, vom Skalenträger 112 radial außen in Axialrichtung überlappt zu werden (siehe insbesondere Fig. 4). Das Einstellmittel 104 weist insbesondere im Axialabschnitt mit glatter Außenfläche ein erstes Anschlagmittel 118 beispielsweise in Form eines Vorsprungs auf, der sich gemeinsam mit dem Einstellmittel (Drehrad) 104 dreht. Der Vorsprung 118 kommt beim Drehen des Einstellmittels 104 mit Erreichen einer bestimmten Drehposition in Kontakt/Anlage mit der Skala, bzw. des Skalenträger 112, der in der Erfindung gemäß diesem Ausführungsbeispiel federelastisch ist. Der Vorsprung 118 drückt demnach beim (Weiter-)Drehen gegen die Skala/Skalenträger 112, der sich somit von der Mittelachse/ Drehachse des Einstellmittels 104 (radial) elastisch wegbewegt, d.h. radial nach außen verdrängt wird, wodurch (mit radial nach außen Drängen des Skalenträgers 112) ein erhöhter Drehwiderstand erzeugt wird. In der Skala/Skalenträger 112 ist auf der dem Einstellmittel 104 und somit auch dem Vorsprung 118 zugewandten Seite ein weiteres Anschlagmittel 120 beispielsweise in Form einer Nut eingebracht oder ausgebildet, die sich parallel zu der Drehachse erstreckt. Der Vorsprung 118 rastet demnach bei einem Weiterdrehen des Einstellmittels 104 (schlagartig) in die Nut 120 ein und die Skala/Skalenträger 112 bewegt sich somit durch den Federdruck schnell bzw. ruckartig in Richtung hin zum Vorsprung 118 (d.h. radial nach innen), wodurch ein akustisches, taktiles, haptisches und/oder teils auch sichtbares Feedback oder Signal (Einrastklicken) an den Benutzer gegeben wird.

Eine einzige Drehung des Einstellmittels 104 lässt den Taster 108 beispielsweise um einen oder zwei Millimeter relativ zu dem Einstellmittel 104 längs des Verstellmittels 106 verfahren, wobei nach jeder Umdrehung (entspricht jedem Millimeter oder nach zwei Millimeter) das erste Anschlagmittel 118 in das weitere (zweite) Anschlagmittel 120 einrastet und somit ein Feedback an den Benutzer gibt, so dass dieser weiß, dass der Taster 108 um einen oder zwei Millimeter verfahren wurde und zwar durch eine entsprechende Drehung des Einstellmittels 104.

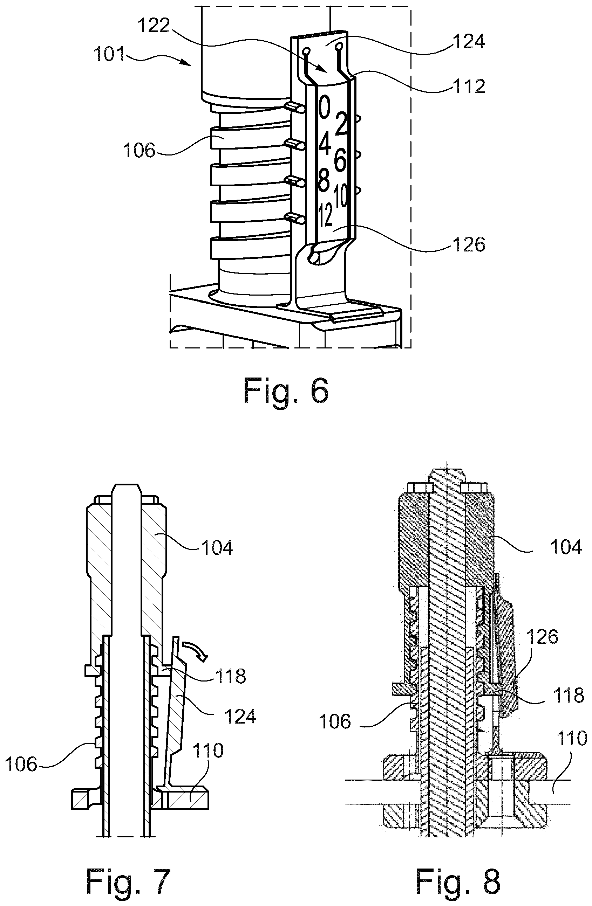

Wie vorstehend bereits ausgeführt wurde, ist es bevorzugt, den Tasterhalter 110 fest mit dem Verstellmittel 106 zu verbinden. In diesem Fall ist die innere Achse/Haltebolzen vorgesehen (siehe insbesondere Fig. 8), auf den das Verstellmittel 106 in Form des Hohlzylinders mit Außengewinde axialverschiebbar und vorzugsweise drehfest aufgesteckt ist. Das Einstellmittel 104 in Form des zuvor beschriebenen Dreh-/Wendelrads ist drehbar aber axialfest am proximalen freien Ende/Endabschnitt der (inneren) Ache gelagert und weist ein Innengewinde auf, das mit dem Außengewinde des Verstellmittels 106 in Schraubeingriff ist. Sobald das Einstellmittel 104 um die (innere) Achse gedreht wird, wird das Verstellmittel 106 in Form des Hohlzylinders axial längs der (inneren) Achse/Haltebolzen bewegt und somit die Distanz zwischen dem Taster 108 und dem Einstellmittel 104 verändert. Alternativ hierzu wäre es aber auch denkbar, das Drehrad fest mit dem Verstellmittel (Wendel) 106 zu verbinden und bei Betätigung des Einstellmittles 104 das Verstellmittel 106 zu drehen. In diesem Fall ist der Tasterhalter 110 in Schraubeingriff mit dem Außengewinde des Verstellmittels 106 und wird bei einer entsprechenden Drehung des Verstellmittels axial verschoben. Als eine Drehsicherung des Tasterhalters 110 könnte beispielsweise der Taster 108 manuell festgehalten oder in einer parallel zum Verstellmittel 106 angeordneten ortsfesten Schiene (nicht gezeigt) längsgeführt werden.

Fig. 5 zeigt die erste Ausführungsform der Ausrichtungsvorrichtung 101 gemäß der Erfindung in einer Frontansicht. Der Aufbau ist analog zu der Figur 4. Das Einstellmittel 104 verschiebt den Tasterhalter 110 über das Verstellmittel/Wendel 106 (diese ist mit dem Tasterhalter 110 und/oder dem Skalenträger 112 fest verbunden) durch Drehung um den (inneren)Haltebolzen, wodurch auch der Taster 108 in dem Tasterhalter 110 mitbewegt wird. An dem Tasterhalter 110 ist die Skala 112 bevorzugt angebracht und ist somit fest mit dem Tasterhalter 110 verbunden. Das erste Anschlagmittel 118 am Einstellmittel 104 kommt in Kontakt mit der Skala/Skalenträger 112 und biegt diesen nach außen, also von der Drehachse weg. Da die Skala/Skalenträger 112 an ihrem/seinem unteren (distalen) Ende an dem Tasterhalter 110 befestigt ist, ist die notwendige Kraft zum nach außen biegen der Skala 112 oben (proximal) niedriger als unten (distal), wo die Skala befestigt ist (infolge Hebelwirkung). Das bedeutet, oben (proximal) wird weniger Kraft benötigt, um die Skala 112 auszulenken als weiter unten (distal). Vorzugsweise ist die Skala/Skalenträger 112 aus Federstahl oder einem anderen Metall mit einer hohen Federsteifigkeit oder Federelastizität. Die Skala/Skalenträger 112 gemäß der Erfindung ist jedoch bevorzugt in zwei serielle Federelemente 124 und 126 unterteilt, die die Kraft in Reihe aufteilen (siehe hierzu Figur 7 und Figur 8). Die Skala/Skalenträger 112 hat dabei vorzugsweise die Form eines Plättchens/ Rechtecks, das an einem Ende mit dem Tasterhalter 110 fest verbunden ist. In der Skala/skalenträger 112 ist ein Einschnitt eingebracht in Form eines umgedrehten U, wodurch das erste Federelement 124 durch den dadurch entstehenden äußeren Skalarahmen gebildet wird und das zweite Federelement 126 durch die dadurch entstandene innere Federzunge gebildet wird, wobei die Federzunge am axial freien Ende (oberes/proximales Ende) des Skalenrahmens an diesem fixiert ist. Durch diese Reihenanordnung der Federelemente 124 und 126 ist die Kraft für ein Wegdrücken des Skalenträgers mittels des ersten Anschlags an dem unteren (distalen) Ende der Skala/Skalenträger im Wesentlichen die gleiche wie die die Kraft für ein Wegdrücken des Skalenträgers mittels des ersten Anschlags an dem oberen (proximalen) Ende der Skala/Skalenträger (Hebelweg zum jeweiligen Schwenkpunkt "unten/oben" bleibt im Wesentlich gleich).

Fig. 6 zeigt die erste Ausführungsform einer Ausrichtungsvorrichtung 101 gemäß der Erfindung in einer Detailansicht. Das Verstellmittel 106 ist mit der Skala 112 über den Tasterhalter 110 fest verbunden. Die Skala 112 ist in anderen Worten ausgedrückt eine Feder 122 mit einem ersten Biege-Federelement 124 und einem zweiten Biege-Federelement 126, das in Reihe zum ersten Federelement geschaltet ist, wobei die Schwenkpunkte der beiden Biegefedern an den voneinander abgewandten Federenden positioniert sind. Durch die U-förmige Aussparung in der Skala 112 verlängert sich der Hebel der gesamten Feder 122, wobei das erste Federelement 124 und das zweite Federelement 126 in Reihe sind. Durch die Aussparung wird das erste Federelement 124 ausgebildet durch zwei Seitenelemente, die die U-förmige Aussparung seitlich einfassen. Um den gewünschten Einrasteffekt und eine akustische Rückmeldung (ein deutlich hörbares Klicken) zu erhalten, ist die Skala 112 im oberen und unteren Bereich federnd ausgelegt, nämlich im oberen Bereich durch das (äußere, rahmenförmige) Federelement 124, im unteren Bereich durch das (innere, zungenförmig) Federelement 126. Das zungenförmige Federelement 126 vermeidet somit einen (linear) ansteigenden Kraftaufwand, wenn das Drehrad/Einstellmittel 104 nach unten gedreht wird, bzw. wenn das Einstellmittel 104 das Verstellmittel 106 nach oben zieht. Das zweite (zungenförmige) Federelement 126 hat den Fixpunkt am oberen/proximalen Ende der Skala 112, wobei sich oben auf die nicht befestigte Seite der Skala 112 an dem Tasterhalter 110 bezieht, und das erste (rahmenförmige) Federelement 124 hat den Fixpunkt auf der unteren/distalen Seite an dem Tasterhalter 110. Im oberen Bereich arbeitet demzufolge der Rahmen 124 als Feder. Je weiter das Verstellmittel 106 zu dem Einstellmittel 104 hin bewegt wird, desto mehr übernimmt die innere Zunge bzw. das Federelement 126 die Federfunktion. Die akustische Rückmeldung erfolgt, wenn das erste Anschlagmittel 118 in das zweite Anschlagmittel 120, das sich in einer der beiden Federelemente 124 oder 126 befindet, einrastet, z.B. pro Umdrehung bei 2mm Gewindesteigung.

Fig. 7 zeigt die erste Ausführungsform der Ausrichtungsvorrichtung 101 gemäß der Erfindung in einer Schnittansicht in einer ersten Position. Die erste Position zeigt das Einstellmittel 104, welches das Verstellmittel 106 umgreift und dieses durch Rotation des Einstellmittels 104 bewegen kann. Das Einstellmittel 104 weist zwei Vorsprünge 118 auf, wobei einer der Vorsprünge gegen das erste Federelement 124 drückt, das an einem Ende an dem Tasterhalter 110 befestigt ist. Das Federelement wird durch den Vorsprung 118 von dem Einstellmittel 104 weggedrückt und biegt sich dadurch radial nach außen.

Fig. 8 zeigt die erste Ausführungsform der Ausrichtungsvorrichtung 101 gemäß der Erfindung in einer Schnittansicht in einer zweiten Position. In der zweiten Position hat das Einstellmittel 104 das Verstellmittel 106 zu sich gezogen, wodurch auch der Tasterhalter 110 mit der Skala darauf näher am Einstellmittel 104 ist. Der Vorsprung 118 befindet sich nun nahe an dem Tasterhalter 110. Durch die U-förmige Aussparung wird nun das zweite Federelement 126 nach außen von dem Einstellmittel 106 weg gedrückt.

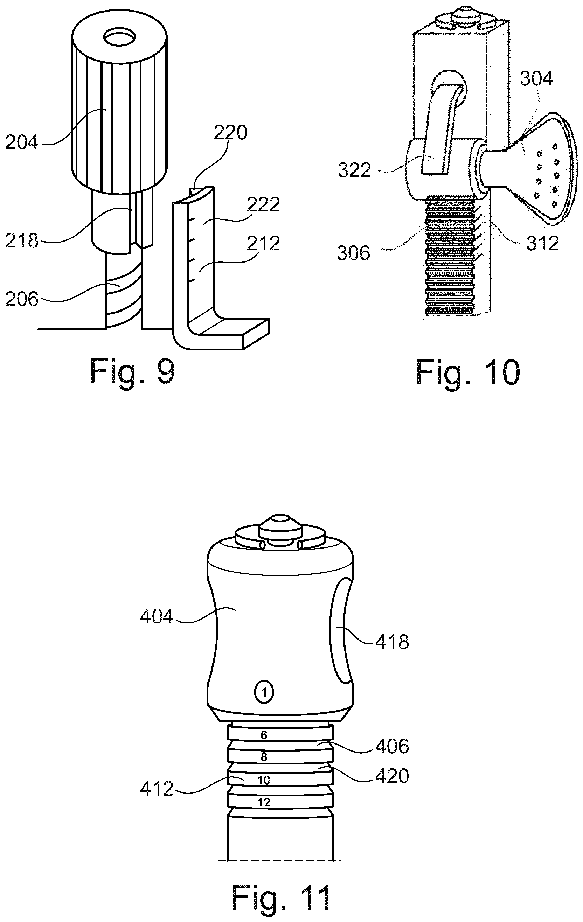

Fig. 9 zeigt eine zweite Ausführungsform einer Ausrichtungsvorrichtung gemäß der Erfindung. In dieser Ausführungsform ist das Einstellmittel 204 drehbar auf dem Verstellmittel 206 aufgebracht und bewegt dieses, in einer Weise wie dies vorstehend bereits beschrieben wurde. In dieser Ausführungsform ist das erste Anschlagmittel 218 an dem Einstellmittel 206 eine Nut, die sich außen an dem Einstellmittel 204 parallel zu der Rotationsachse des Einstellmittels 204 erstreckt. An der Skala 212, die analog zu dem ersten Ausführungsbeispiel eine Feder 222 ist und eine Federelastizität aufweist, ist das zweite Anschlagmittel 220 in Form eines Vorsprungs ausgebildet.

Fig. 10 zeigt eine dritte Ausführungsform einer Ausrichtungsvorrichtung gemäß der Erfindung. Ein Einstellmittel 304 ist in Form eines Zahnrades ausgebildet, das in das Verstellmittel 306 in Form einer Oberfläche mit Zähnen eingreift. An der Seite des Verstellmittels 306 ist eine Skala ausgebildet. Eine Feder 322 weist auf einer dem Einstellmittel 304 zugewandten Seite einen Vorsprung auf (nicht dargestellt) der in die Zähne des Einstellmittels 304 eingreift und somit ein akustisches und haptisches Feedback an den Benutzer gibt, wenn dieser das Einstellmittel 304 dreht.

Fig. 11 zeigt eine vierte Ausführungsform einer Ausrichtungsvorrichtung gemäß der Erfindung. Das Einstellmittel 404 hat in einer Innenseite eine federvorgespannte Kugel (nicht dargestellt) als erstes Anschlagmittel 418. Das Verstellmittel 406 weist Einkerbungen auf, die als zweites Anschlagmittel 420 dienen. An dem Verstellmittel ist an den Vorsprüngen zwischen den Einkerbungen eine Skala 412 aufgebracht.

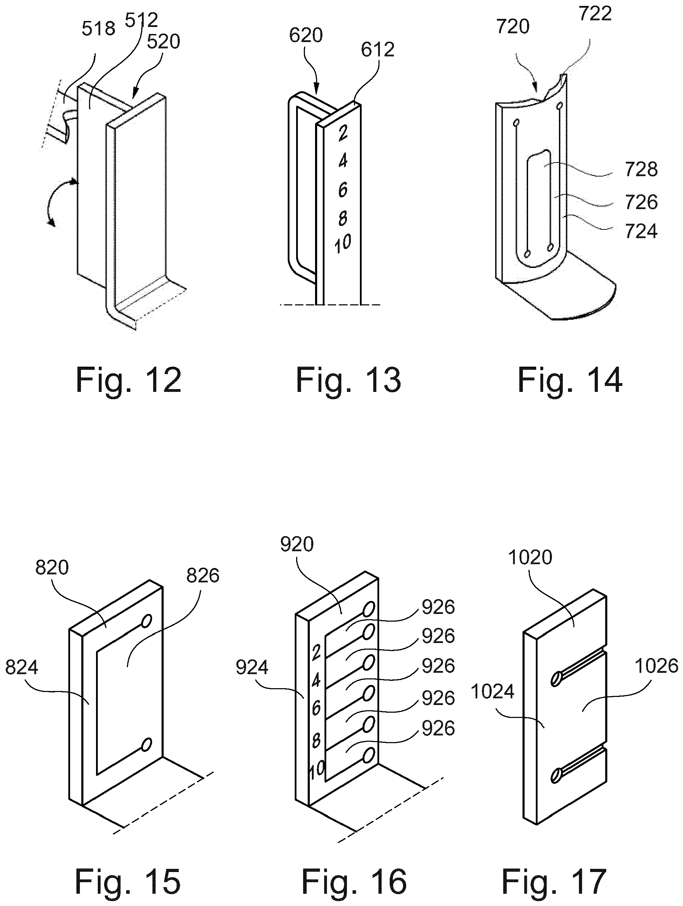

Fig. 12 zeigt eine fünfte Ausführungsform einer Ausrichtungsvorrichtung gemäß der Erfindung. Eine Skala 512 ist dabei mit einem T-förmigen Querschnitt ausgebildet. In anderen Worten weist eine rechteckförmige Fläche an seiner langen Mittelachse eine weitere rechteckförmige Fläche senkrecht dazu auf. Diese zweite Fläche oder Fahne ist ein federelastisches Anschlagmittel 520 in Form eines Federplättchens, das in ein zweites Anschlagmittel 518 (z.B. einer Nut) an einem Einstellmittel (nicht dargestellt) eingreift bzw. einrasten kann.

Fig. 13 zeigt eine sechste Ausführungsform einer Ausrichtungsvorrichtung gemäß der Erfindung. Eine Skala 612 ist dabei mit einem Draht als Anschlagmittel 520 ausgebildet. In anderen Worten weist eine rechteckförmige Fläche an seiner langen Mittelachse einen abgewinkelten, federelastischen Draht/ Federdraht dazu auf, der an zwei Enden mit der Skala 612 verbunden ist. Dieser Draht ist somit ein federelastisches Anschlagmittel 620 das in ein zweites Anschlagmittel (z.B. einer Nut) an einem Einstellmittel (nicht dargestellt) eingreift bzw. einrasten kann.

Fig. 14 zeigt eine siebte Ausführungsform einer Ausrichtungsvorrichtung gemäß der Erfindung. Die Skala bzw. Feder 722 ist dabei analog zu der ersten Ausführungsform mit der Ausnahme, dass die Skala ein drittes Federelement 728 in Reihe zu einem ersten Federelement 724 und einem zweiten Federelement 726 aufweist. Dabei ist das dritte Federelement 726 wieder in Form eines U ausgebildet, dass genau um 180° zu dem ersten U versetzt ist, und hat ihren Fixpunkt wieder an dem oberen Ende. Somit ist der Kraftaufwand noch linearer als im ersten Ausführungsbeispiel

Fig. 15 zeigt eine achte Ausführungsform einer Ausrichtungsvorrichtung gemäß der Erfindung. Dabei ist das zweite Anschlagmittel 820 ähnlich der ersten Ausführungsform ausgestaltet, mit dem Unterschied, dass das zweite Federelement 826 U-förmig in einem Winkel von 90° gedreht in dem ersten Federelement 824 angebracht ist, bzw. den Fixpunkt in einem Winkel von 90° zu dem unteren Ende aufweist.

Fig. 16 zeigt eine neunte Ausführungsform einer Ausrichtungsvorrichtung gemäß der Erfindung. Das zweite Anschlagmittel 920 ist in dieser Ausführungsform mit eine Vielzahl von zweiten Federelementen 926 ausgestatte, die wie in der achten Ausführungsform ausgebildet sind. Die Vielzahl der zweiten Federelement 926 sind parallel von unten nach oben angeordnet und werden von dem ersten Federelement 924 eingerahmt.

Fig. 17 zeigt eine zehnte Ausführungsform einer Ausrichtungsvorrichtung gemäß der Erfindung. Das zweite Anschlagmittel 1020 weist in dieser Ausführungsform wenigstens zwei Einschnitte auf, die parallel zu dem unteren Ende des Anschlagmittels 1020 verlaufen, an dem dieses an dem Tasterhalter (nicht dargestellt) befestigt ist. Somit wird das Anschlagmittel 1020, das auch die Skala bildet, an verschiedenen Stellen radial ausgelenkt.

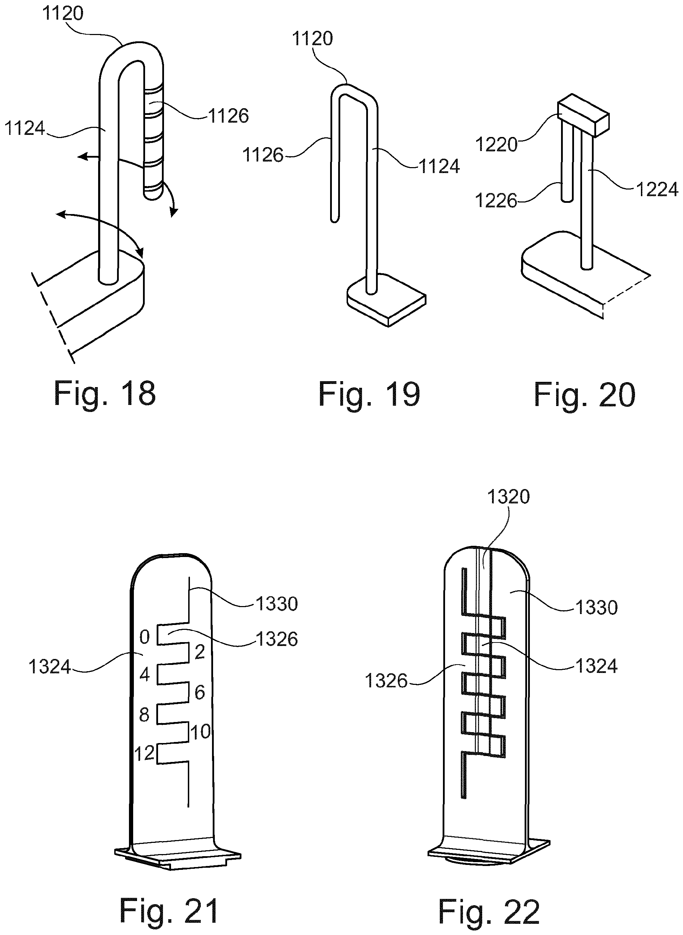

Fig. 18 zeigt eine elfte Ausführungsform einer Ausrichtungsvorrichtung gemäß der Erfindung mit seitlicher Belastung. Das Anschlagmittel 1120 besteht aus einem Federdraht in Form eines Hakens/ eines U der/ das an einem Ende an dem Tasterhalter befestigt ist. Ein erstes Federelement 1124 hat den Fixpunkt an dem Tasterhalter, ein zweites Federelement 1126 hat den Fixpunkt an dem oberen Ende des ersten Federelements 1124. Das Anschlagmittel 1120 kann in Rotationsrichtung eines nicht dargestellten drehbaren Einstellmittels ausgelenkt werden wie die Pfeile darstellen.

Fig. 19 zeigt die elfte Ausführungsform einer Ausrichtungsvorrichtung gemäß der Erfindung mit frontaler Belastung. Zusätzlich zu der in der in Figur 18 dargestellten Rotations-Auslenkung des ersten Federelements 124 und/oder zweiten Federelements 126 kann das zweite Anschlagmittel 1120 zudem eine radiale Ausrichtung erfahren, wie durch die Pfeile in Figur 19 dargestellt.

Fig. 20 zeigt eine zwölfte Ausführungsform einer Ausrichtungsvorrichtung gemäß der Erfindung mit frontaler Belastung. Der Aufbau der zwölften Ausführungsform ist analog zu der elften Ausführungsform, mit der Ausnahme, dass das Anschlagsmittel 1220 nicht aus einem einzigen U-förmigen Draht besteht, sondern, dass das erste Federelement 1224, in Form eines geraden Federdrahtes, an seinem oberen Ende mit einer Platte oder dergleichen mit dem zweiten Federelement 1226 verbunden ist, das parallel zu dem ersten Federelement 1224 in Form eines geraden Federdrahtes ausgebildet ist.

Fig. 21 zeigt eine dreizehnte Ausführungsform einer Ausrichtungsvorrichtung gemäß der Erfindung in Frontansicht. Die Skala weist in dieser Ausführungsform einen rechteckförmigen Schnitt 1330 auf. Der rechteckförmige Schnitt 1330 führt von der Nähe des oberen Endes der Skala zu der Nähe des unteren Endes der Skala. Die Skala wird durch den rechteckförmigen Schnitt 1330 in ein erstes Federelement 1324 und ein zweites Federelement 1326 geteilt. Diese Ausführungsform erlaubt ein akustisches, visuelles und taktiles Feedback nach dem Prinzip der Drehorgel. Wenn eine rechteckförmige Fläche ausgelenkt wird durch einen Vorsprung eines Einstellmittels (nicht dargestellt) federt das andere Federelement zurück und andersrum.

Fig. 22 zeigt die dreizehnte Ausführungsform einer Ausrichtungsvorrichtung gemäß der Erfindung in Rückansicht. An der Rückseite der Skala ist als zweites Anschlagmittel eine Nut 1320 eingebracht in vom oberen Ende zu dem unteren Ende verläuft und durch das erste Federelement 1324 und das zweite Federelement 1326 verläuft. Die Nut verläuft auch durch den rechteckfömigen Schnitt.

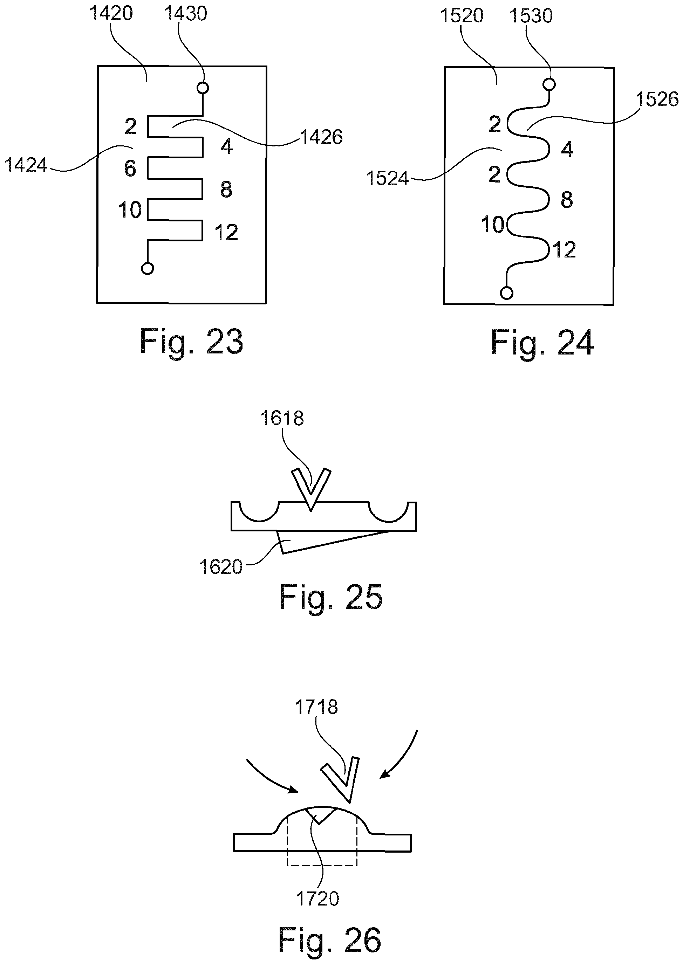

Fig. 23 zeigt eine vierzehnte Ausführungsform einer Ausrichtungsvorrichtung gemäß der Erfindung, Diese Ausführungsform ist analog, zu der dreizehnten Ausführungsform mit dem einzigen Unterschied dass diese Ausführungsform auf der Rückseite keine Nut aufweist und ohne Nut als zweites Anschlagmittel 1420 dient. Auch in dieser Ausführungsform trennt ein rechteckförmiger Schnitt 1430 das Anschlagmittel in ein erstes Federelement 1424 und ein zweites Federelement 1426 von oben nach unten.

Fig. 24 eine fünfzehnte Ausführungsform einer Ausrichtungsvorrichtung gemäß der Erfindung. Diese Ausführungsform ist analog zu der dreizehnten oder vierzehnten Ausführungsform mit dem Unterschied, dass ein Schnitt 1530 nicht rechteckförmig ist sondern die Form einer Welle aufweist und ein zweites Anschlagmittel 1520 in ein erstes Federelement 1524 und ein zweites Federelement 1526 unterteilt.

Fig. 25 eine sechzehnte Ausführungsform einer Ausrichtungsvorrichtung gemäß der Erfindung. Diese Ausführungsform ist anwendbar auf alle vorhergehenden Ausführungsformen die ein weiteres Federelement/ zweites Federelement, das durch einen U-förmigen Schnitt von dem ersten Federelement getrennt ist, in der Skala aufweisen. Dabei wird ein erstes Anschlagmittel 1618 durch Drehung eines Einstellmittels an ein zweites Anschlagmittel 1620 gebracht. Das zweite Anschlagmittel 1620 bewegt sich radial von einer Drehachse des drehbaren Einstellmittels radial weg. Das erste Anschlagmittel 1618 stößt an einen Rahmen des zweiten Anschlagmittels 1620. Das zweite Anschlagmittel 1620 ist dabei durch einen Schnitt, vorzugsweise einem U förmigen, so vom Rest der Skala getrennt, dass es an einer Seite ausgelenkt werden kann.

Fig. 26 eine siebzehnte Ausführungsform einer Ausrichtungsvorrichtung gemäß der Erfindung. Diese Ausführungsform ist anwendbar auf alle vorhergehenden Ausführungsformen die ein weiteres Federelement/ zweites Federelement, das durch einen U-förmigen Schnitt von dem ersten Federelement getrennt ist, in der Skala aufweisen. In dieser Ausführungsform weist das zweite Anschlagmittel 1720 eine abgerundete Oberfläche um eine Nut herum auf, so dass das erste Anschlagmittel 1718 sanfter in Kontakt mit diesem kommt.

Zusammengefasst betrifft die vorliegende Erfindung eine Antast-/Ausrichtungsvorrichtung, die zum Ausrichten einer Sägeschablone ein Einstellmittel, ein Verstellmittel mit einer profilierten Einkerbung und einen Taster aufweist, der relativ zu dem Einstellmittel bewegbar ist, eine Skala zum Messen einer Höhe des Einstellmittels relativ zu dem Verstellmittel, wenigstens ein erstes Anschlagmittel, das an dem Einstellmittel ausgeformt ist, wenigstens ein zweites Anschlagmittel das an der Skala ausgeformt ist oder als Skala dient und das vorgesehen und angepasst ist als Widerstand für das erste Anschlagmittel zu fungieren, wobei das zweite Anschlagmittel und/oder das erste Anschlagmittel das jeweils andere Anschlagmittel federnd auslenkt.

### Bezugszeichenliste

- 1, 101: Stylus
- 2: Tibiakopf
- 4, 104, 204, 304, 404: Einstellmittel
- 6, 106, 206, 306, 406: Verstellmittel
- 8, 108: Taster
- 10, 110: Tasterhalter
- 12,112,212,312,412,512,612: Skala
- 14: Sägeschablone
- 16: extramedullärer Halter
- 118, 218, 418, 518, 1618, 1718: erstes Anschlagmittel
- 120, 220, 420, 520, 620, 720, 820, 920, 1020, 1120, 1220, 1320, 1420, 1520, 1620, 1720: zweites Anschlagmittel
- 122,222,322,722: Feder
- 124, 724, 824, 924, 1024, 1124, 1224, 1324, 1424, 1524: erstes Federelement
- 126, 726, 826, 926, 1126, 1226, 1326, 1426, 1526: zweites Federelement
- 728: drittes Federelement
- 1330, 1430, 1530: Schnitt

## Patentansprüche

1. Ausrichthilfe für eine tibiale Resektionsführung mit oder bestehend aus einer Antastvorrichtung (1) aufweisend:
- ein wendel- oder zahnstangenförmiges Verstellelement (6),
- einen Taster (8), der an dem Verstellelement (6) gehalten ist,
- ein wendelrad- oder zahnradförmiges Einstellelement (4), welches mit dem Verstellelement (6) in Eingriff oder gekoppelt ist, um durch manuelle Betätigung des Einstellelements (4) den Taster (8) mittels des Verstellelements (6) zu bewegen,
- ein mit dem Taster (8) oder dem Verstellelement (6) gekoppeltes sowie längs des Verstellelements (6) sich erstreckender Skalenträger (12), der mit Referenz auf eine Markierung am Einstellelement (4), am Taster (8) oder am Verstellelement (6) eine aktuelle Position des Tasters (8) vorzugsweise mit Bezug zum Einstellelement (4) angibt,
- zumindest ein erstes Rast- oder Anschlagteil (118) und
- zumindest ein zweites Rast- oder Anschlagteil (120),
- wobei
- das erste Rast- oder Anschlagsteil (118) und/oder das zweite Rast- oder Anschlagsteil (120) federelastisch nachgiebig oder federelastisch nachgiebig gelagert ist, sodass
- das erste und zweite Rast- oder Anschlagteil (118, 120) bei einer Betätigung des Einstellelements (4) in einer bestimmten Relativdrehstellung oder innerhalb eines bestimmten Relativstellungsbereichs zwischen dem Skalenträger (12) und dem Einstellelement (4) das erste Rast- oder Anschlagteil (118) und/oder das zweite Rast- oder Anschlagsteil (120) das jeweils andere Anschlagsteil federnd auslenkt und dass
- bei einer Weiterbetätigung des Einstellelements (4) das erste und/oder zweite Anschlagteil (118, 120) vorzugsweise schlagartig in einen Rast- oder Anschlagseingriff miteinander zurückfedert,
**dadurch gekennzeichnet, dass**
- das zumindest eine erste Rast- oder Anschlagteil (118) am Einstellelement (4) ausgeformt ist und
das zumindest eine zweite Rast- oder Anschlagteil (120) am Skalenträger (12) ausgeformt oder durch den Skalenträger (12) gebildet ist.

2. Ausrichthilfe nach Anspruch 1, **dadurch gekennzeichnet, dass** beim Zurückfedern das zweite Anschlagmittel (120) und/oder das erste Anschlagmittel (118) in das jeweils andere Anschlagmittel einrastet und dabei ein haptisches, taktiles, akustisches und/oder sichtbares Feedback oder Signal gegeben wird oder erzeugt.

3. Ausrichthilfe nach einem der vorstehenden Ansprüche 1 bis 2, **dadurch gekennzeichnet, dass** das Verstellelement (6) ein Außengewinde vorzugsweise in Form einer Wendel oder eine Oberfläche mit Zähnen vorzugsweise in Form einer Zahnstange aufweist oder ist.

4. Ausrichthilfe nach einem der vorstehenden Ansprüche 1 bis 3, **dadurch gekennzeichnet, dass** das Einstellelement (4) ein Drehrad vorzugsweise mit Innengewinde oder ein Zahnrad ist.

5. Ausrichthilfe nach einem der vorstehenden Ansprüche 1 bis 4, **dadurch gekennzeichnet, dass** das erste Anschlagelement (118) eine Nut oder ein Vorsprung ist und das zweite Anschlagelement (120,) entsprechend umgekehrt ein Vorsprung oder eine Nut ist.

6. Ausrichthilfe nach einem der vorstehenden Ansprüche 1 bis 4, **dadurch gekennzeichnet, dass** das der Skalenträger (12) einstückig mit dem Taster (8) ausgeformt ist oder als ein einziges Bauteil ausgebildet und am Taster (8) montiert ist.

7. Ausrichthilfe nach einem der vorstehenden Ansprüche 1 bis 6, **dadurch gekennzeichnet, dass** das der Skalenträger (12) wenigstens zwei Federelemente (124, 126) in Reihe aufweist oder aus diesen besteht, wobei der eine Federarm (124) an seinem distalen Ende mit dem Taster (8) gekoppelt und sich in Richtung proximal erstreckt und der andere Federarm (126) mit proximalen Ende oder Endabschnitt des einen Federarms (124) verbunden ist und sich in Richtung distal erstreckt.

8. Ausrichthilfe nach einem der vorstehenden Ansprüche 1 bis 7, **gekennzeichnet durch** einen Haltebolzen, der dazu vorgesehen und ausgebildet ist, an seinem distalen Endabschnitt mit einer Sägeschablone (14) verbunden zu werden, der als drehfeste Axialschiebeführung für das als Hohlzylinder mit Außengewinde ausgebildete Verstellelement (6) dient und der an seinem freien, proximalen Endabschnitt das Einstellelement (4) in Form eines Wendelrads mit Innengewinde drehbar und axialfest lagert, welches mit dem Außengewinde des Hohlzylinders (6) in Schraubeingriff ist, um bei einer Drehbetätigung den Hohlzylinder (6) längs des Haltebolzens zu bewegen.

9. Ausrichthilfe nach Anspruch 8, **dadurch gekennzeichnet, dass** das Wendelrad (4) einen proximalen Betätigungssabschnitt und distalen Axialabschnitt hat, der an seiner radialen Außenseite vom Skalenträger (12) über eine Axialstrecke hinweg überlappt ist, wobei sich die Axialstrecke in Abhängigkeit des Betätigungsbetrags des Wendelrads (4) verändert.

10. Ausrichthilfe nach Anspruch 9, **dadurch gekennzeichnet, dass** das erste Rast- oder Anschlagsteil (118) in dem distalen Axialabschnitt des Wendelrads (4) ausgebildet ist.

## Claims

1. An orientation apparatus for a tibial resection guide comprising or consisting of a probing device (1) having:
- a helical-shaped or gear-rack shaped adjustment element (6),
- a feeler (8) which is held on the adjustment element (6),
- a helical-wheel shaped or gear-shaped setting element (4) which is engaged with or coupled to the adjustment element (6) in order to move the feeler (8) via the adjustment element (6) by manual actuation of the setting element (4),
- a scale carrier (12) coupled to the feeler (8) or the adjustment element (6) and extending along the adjustment element (6), wherein the scale carrier (12) indicates a current position of the feeler (8), preferably with respect to the setting element (4), with reference to a marking on the setting element (4), on the feeler (8) or on the adjustment element (6),
- at least one first latch or stop member (118) and
- at least one second latch or stop member (120),
- wherein
- the first latch or stop member (118) and/or the second latch or stop member (120) is spring-elasticly yielding or mounted in a spring-elasticly yielding manner, so that
- the first and the second latch or stop member (118, 120), upon actuation of the setting element (4) in a specific relative rotational position or within a specific relative position range between the scale carrier (12) and the setting element (4), the first latch or stop member (118) and/or the second latch or stop member (120) deflects the respective other stop member in a spring-elastic manner, and in that
- upon further actuation of the setting element (4), the first and/or second stop member (118, 120) springs back, preferably abruptly, into a latch or stop engagement with each other,
**characterized in that**
- the at least one first latch or stop member (118) is formed on the setting element (4), and
the at least one second latch or stop member (120) is formed on the scale carrier (12) or is formed by the scale carrier (12).

2. The orientation apparatus according to claim 1, **characterized in that** during spring-back the second stop means (120) and/or the first stop means (118) engages the respective other stop means and thereby a haptic, tactile, acoustic and/or visible feedback or signal is given or generated.

3. The orientation apparatus according to one of the preceding claims 1 to 2, **characterized in that** the adjustment element (6) comprises or is an external thread preferably in the form of a helix or a surface with teeth preferably in the form of a gear rack.

4. The orientation apparatus according to one of the preceding claims 1 to 3, **characterized in that** the setting element (4) is a rotary wheel, preferably with an internal thread, or a gear wheel.

5. The orientation apparatus according to one of the preceding claims 1 to 4, **characterized in that** the first stop element (118) is a groove or a projection and the second stop element (120) is correspondingly reversely a projection or a groove.

6. The orientation apparatus according to one of the preceding claims 1 to 4, **characterized in that** the scale carrier (12) is formed in one piece with the feeler (8) or is formed as a single component and is mounted on the feeler (8).

7. The orientation apparatus according to one of the preceding claims 1 to 6, **characterized in that** the scale carrier (12) comprises or consists of at least two spring elements (124, 126) in series, wherein one spring arm (124) is coupled at its distal end to the feeler (8) and extends in a proximal direction and the other spring arm (126) is coupled to a proximal end or end portion of the one spring arm (124) and extends in a distal direction.

8. The orientation apparatus according to one of the preceding claims 1 to 7, **characterized by** a retaining bolt which is provided and formed to be connected at its distal end portion to a saw template (14), which serves as a rotationally fixed, axial sliding guide for the adjustment element (6) formed as a hollow cylinder with an external thread and which, at its free, proximal end portion, supports the setting element (4) in the form of a helical wheel with an internal thread in a rotatable and axially fixed manner, which is in threaded engagement with the external thread of the hollow cylinder (6), in order to move the hollow cylinder (6) along the retaining bolt during rotary actuation.

9. The orientation apparatus according to claim 8, **characterized in that** the helical wheel (4) has a proximal actuation portion and a distal axial portion, which is overlapped at its radially outer side by the scale carrier (12) over an axial distance, wherein the axial distance varies depending on the actuation amount of the helical wheel (4).

10. The orientation apparatus according to claim 9, **characterized in that** the first latch or stop member (118) is formed in the distal axial portion of the helical wheel (4).

## Revendications

1. Aide à l'alignement pour un guidage de résection tibial avec ou consistant en un dispositif de palpage (1) présentant :
- un élément de déplacement (6) en forme de spirale ou de crémaillère,
- un palpeur (8) qui est maintenu au niveau de l'élément de déplacement (6),
- un élément de réglage (4) en forme de roue hélicoïdale ou de roue dentée, lequel est en prise ou couplé à l'élément de déplacement (6) afin de déplacer le palpeur (8) au moyen de l'élément de déplacement (6) par l'actionnement manuel de l'élément de réglage (4),
- un support de graduation (12) couplé au palpeur (8) ou à l'élément de déplacement (6) ainsi que s'étendant le long de l'élément de déplacement (6) qui indique, avec référence sur un marquage sur l'élément de réglage (4), sur le palpeur (8) ou sur l'élément de déplacement (6), une position actuelle du palpeur (8), de préférence en ce qui concerne l'élément de réglage (4),
- au moins une première partie d'encliquetage ou de butée (118) et
- au moins une seconde partie d'encliquetage ou de butée (120),
- dans laquelle
- la première partie d'encliquetage ou de butée (118) et/ou la seconde partie d'encliquetage ou de butée (120) est flexible comme un ressort ou est logée de manière flexible comme un ressort de sorte que
- la première et seconde partie d'encliquetage ou de butée (118, 120), lors d'un actionnement de l'élément de réglage (4) dans une position rotative relative déterminée ou dans une plage de position relative déterminée entre le support de graduation (12) et l'élément de réglage (4), la première partie d'encliquetage ou de butée (118) et/ou la seconde partie d'encliquetage ou de butée (120) dévie élastiquement l'autre partie de butée respectivement et que
- lors d'une suite d'actionnement de l'élément de réglage (4), la première et/ou seconde partie de butée (118, 120) rebondit de préférence soudainement dans une prise d'encliquetage ou de butée l'une avec l'autre,
**caractérisée en ce que**
- l'au moins une première partie d'encliquetage ou de butée (118) est façonnée au niveau de l'élément de réglage (4) et
l'au moins une seconde partie d'encliquetage ou de butée (120) est façonnée sur le support de graduation (12) ou est formée par le support de graduation (12).

2. Aide à l'alignement selon la revendication 1, **caractérisée en ce que**, lors du rebondissement, le second moyen de butée (120) et/ou le premier moyen de butée (118) s'encliquète dans l'autre moyen de butée respectif et un retour d'informations ou signal haptique, tactile, acoustique et/ou visible est alors donné ou généré.

3. Aide à l'alignement selon l'une quelconque des revendications précédentes 1 à 2, **caractérisée en ce que** l'élément de déplacement (6) présente ou est un filet extérieur, de préférence sous la forme d'une spirale ou une surface avec des dents, de préférence sous la forme d'une crémaillère.

4. Aide à l'alignement selon l'une quelconque des revendications précédentes 1 à 3, **caractérisée en ce que** l'élément de réglage (4) est une roue rotative, de préférence avec un filet intérieur ou une roue dentée.

5. Aide à l'alignement selon l'une quelconque des revendications précédentes 1 à 4, **caractérisée en ce que** le premier élément de butée (118) est une rainure ou une saillie et le second élément de butée (120) est une saillie ou une rainure de manière inversée correspondante.

6. Aide à l'alignement selon l'une quelconque des revendications précédentes 1 à 4, **caractérisée en ce que** le support de graduation (12) est façonné d'un seul tenant avec le palpeur (8) ou est réalisé comme un composant unique et est monté au niveau du palpeur (8).

7. Aide à l'alignement selon l'une quelconque des revendications précédentes 1 à 6, **caractérisée en ce que** le support de graduation (12) présente au moins deux éléments de ressort (124, 126) en série ou consiste en ceux-ci, dans laquelle l'un bras de ressort (124) est couplé à son extrémité distale au palpeur (8) et s'étend en direction proximale et l'autre bras de ressort (126) est relié à l'extrémité proximale ou à la section d'extrémité de l'un bras de ressort (124) et s'étend en direction distale.

8. Aide à l'alignement selon l'une quelconque des revendications précédentes 1 à 7, **caractérisée par** un boulon de retenue qui est prévu et réalisé afin d'être relié au niveau de sa section d'extrémité distale à un gabarit de sciage (14) qui sert de guidage coulissant axial ne pouvant pas tourner pour l'élément de déplacement (6) réalisé comme cylindre creux avec un filet extérieur et qui loge de manière rotative et fixe, axialement au niveau de sa section d'extrémité libre proximale, l'élément de réglage (4) sous la forme d'une roue hélicoïdale avec un filet intérieur qui est en prise par vissage avec le filet extérieur du cylindre creux (6) afin de déplacer lors d'un actionnement rotatif le cylindre creux (6) le long du boulon de retenue.

9. Aide à l'alignement selon la revendication 8, **caractérisée en ce que** la roue hélicoïdale (4) présente une section d'actionnement proximale et une section axiale distale qui est recouverte au niveau de son côté extérieur radial par le support de graduation (12) au-delà d'un parcours axial, dans laquelle le parcours axial se modifie en fonction de la quantité d'actionnement de la roue hélicoïdale (4).

10. Aide à l'alignement selon la revendication 9, **caractérisée en ce que** la première partie d'encliquetage ou de butée (118) est réalisée dans la section axiale distale de la roue hélicoïdale (4).
